# EUROPEAN PATENT SPECIFICATION

(11) **EP 0 635 577 B1**
(45) Date of publication and mention of the grant of the patent: **27.08.2003**
(21) Application number: 94109776.8
(22) Date of filing: 24.06.1994
(51) Int. Cl.: C12Q 1/04

(54) **Screen for inhibitors of melanin biosynthesis**
Screen für Inhibitoren von Melanin-Biosynthese
Screen pour inhibiteurs de la biosynthèse de mélanine

(30) Priority: 23.07.1993 US 96554
(43) Date of publication of application: 25.01.1995
(73) Proprietor: BASF AKTIENGESELLSCHAFT, 67056 Ludwigshafen (DE)
(72) Inventor: Brindle, Frances Harriet Elizabeth, Hopewell, New Jersey 08525 (US); Froeliger, Eunice Harriet, Hopewell, New Jersey 08525 (US)
(74) Representative: Wächtershäuser, Günter, Prof. Dr.

(56) References cited:
- DATABASE WPI Section Ch, Week 9207 Derwent Publications Ltd., London, GB; Class C07, AN 92-055082 XP002001600 & SU-A-1 635 094 (GOSAGROPROM PHYTOPA) , 15 March 1991
- DATABASE WPI Section Ch, Week 8819 Derwent Publications Ltd., London, GB; Class C02, AN 88-129521 XP002001601 & JP-A-63 072 610 (NIHON TOKUSHU NOYAKU SEI) , 2 April 1988
- DATABASE WPI Section Ch, Week 9215 Derwent Publications Ltd., London, GB; Class A93, AN 92-120219 XP002001602 & JP-A-04 063 868 (DAIICHI KOGYO SEIYAKU) , 28 February 1992
- DATABASE BIOSIS BIOSCIENCES INFORMATION SERVICE, PHILADELPHIA, PA, US AN 7166246, TYUTEREV ET AL.: "Pseudofungicides screening mechanism and application to plant protection" XP002001599 & PRIKLADNAYA BIOKHIMIYA I MIKROBIOLOGIYA, vol. 25, no. 3, 1989, pages 405-416,

## Description

### Technical Field

This invention relates to a fungal screen for inhibitors of melanin biosynthesis, particularly for fungicides that inhibit the biosynthesis of 1,8-dihydroxynaphthalene melanin.

### Background of the Invention

Found in animals, plants, and microorganisms, melanins are biological pigments responsible for the dark color of skin, hair, feathers, fur, and insect cuticle. A number of these diverse macromolecules have been identified and shown to have a polymeric nucleus containing quinone, hydroquinone, and/or semiquinone moieties (reviewed by Bell, A.A., and Wheeler, M.H., *Ann. Rev. Phytopathol. 24:* 411-51 (1986)). Black pigments in animals, for example, are formed from DOPA (3,4-dihydroxyphenylalanine) melanin, biosynthesized in a pathway involving the oxidation of tyrosine by tyrosinase. Red pigments in animals are formed by the polymerization of cysteinyl DOPA, which is the product of cysteine reacted with DOPA quinone, and yellow and brown pigments in animals are mixed polymers formed from both DOPA and cysteinyl DOPA. Animal melanins are synthesized and maintained almost totally as melanosomes and granules within the cytoplasm of specialized cells.

In contrast to animal melanins, fungal melanins generally exhibit a different pattern of polymer deposition, occurring either in cell walls or as extracellular polymers-formed in the medium around fungal cells. Extracellular melanins formed by fungi from tyrosine or tyrosine-containing protein hydrolysates are undoubtedly similar to the DOPA melanins; tyrosinases from mushrooms and *Neurospora crassa* have been thoroughly characterized, and tyrosinase has also been confirmed in other fungi, bacteria, and Actinomycetes (*ibid*.). Melanins in cell walls of Basidiomycotina, on the other hand, are derived from glutaminyl-3,4-dihydroxybenzene (GDHB) or catechol as the immediate phenolic precursor of the melanin polymer. In the Ascomycotina and related Deuteromycotina, the dark-brown to black melanins in cell walls are generally synthesized from the pentaketide pathway in which 1,8-dihydroxynaphthalene (DHN) is the immediate precursor of the polymer.

Melanins have the unique capability of absorbing light over a broad range of wavelengths (∼200 to 2400 nm). In man, the pigment serves as an excellent screen against the untoward effects of solar ultraviolet radiation. In fungi, melanins may be essential for protection of the organism against ultraviolet irradiation as well as radio waves, desiccation, and temperature extremes (Bell and Wheeler, cited above, page 430). The most important role of fungal melanin, however, probably is protecting against hydrolytic enzymes (*id*., page 432). Most antagonists that cause lysis of fungal cells also produce chitinase and glucanases. Generally, the ability of this mixture of enzymes to hydrolyze fungal cell walls is inversely related to the melanin content of the walls. Melanins may also give structural rigidity to complex fungal structures (*id*., page 436).

Fungal melanins are also implicated in the process of pathogenesis for several plant pathogenic fungi including *Magnaporthe grisea,* the causal agent of rice blast disease. A conidium germinates and the tip of the elongating germ tube swells to form a specialized infection structure, the appressorium. Melanization of the appressorium is crucial for subsequent penetration into host epidermal tissue. If the biosynthesis of melanin is blocked by mutation or chemical inhibition, penetration fails and disease is prevented. A block in melanin biosynthesis is demonstrated by distinctive color differences from wild-type pigmentation.

In an albino mutant of *M. grisea,* the melanin biosynthetic pathway is blocked prior to formation of 1,3,6,8-tetrahydroxynaphthalene (1,3,6,8-THN). The rosy mutant is blocked at the conversion of scytalone to 1,3,8-trihydroxy-naphthalene (1,3,8-THN), and the tan-colored buff mutant results from a block between 1,3,8-THN and vermelone. Each of these mutants form appressoria on host tissue but fail to penetrate and cause disease. The chemical inhibitor tricyclazole specifically affects the conversion of 1,3,8-THN to vermelone, and at higher concentrations inhibits the pathway between 1,3,6,8-THN and scytalone. Tricyclazole is used to prevent rice blast in other countries, notably Japan, but is not registered for use in the United States. Specific inhibitors of enzymes required for other steps in the pathway have yet to be found.

### Summary of the Invention

It is an object of this invention to provide a screening test for the identification of agents which inhibit melanin biosynthesis for a variety of agricultural and medical uses.

It is a further and more specific object of this invention to identify agents of this type which exhibit antifungal activity.

These and other objects are accomplished by the present invention, which provides a screening test for the identification of agents that inhibit melanin synthesis. In the practice of the method, test samples are incubated in a culture of a fungus that produces melanin. Agents that are positive in the test produce altered pigmentation in the fungal growth.

In the practice of this invention's method for screening for the presence or absence of melanin synthesis inhibition by a test sample, the test sample is added to a culture or culture area of a melanin-producing fungus, e.g., a fungus that produces dihydroxynaphthalene melanin. Especially preferred are DHN melanin-producing fungi that pigment either in the dark or in the light. The culture is incubated with the test sample for such time under such conditions sufficient to observe cell growth and pigmentation in a corresponding culture or culture area containing no test sample. Preferred screening tests further employ, as a positive control, a known melanin synthesis inhibitor such as pyroquilon or tricyclazole. Melanin synthesis inhibition is determined by observation of alterations in pigmentation in the culture or culture area containing the test sample and alterations in pigmentation in the culture or culture area containing the control.

In a particularly preferred embodiment, *Cladosporium cucumerinum* is grown in a solidified medium in a transparent plate or dish, so that test samples and positive controls are observed visually and simultaneously as regions of the same culture. Tricyclazole is employed as a control. The cultures are grown in the dark or in the light; light is preferred in one embodiment. Actives produce a halo of white, tan or red tones around the test sample in the olive-green lawn of the culture; pigmentation changes are conveniently observed on the underside of the plate or dish.

In some embodiments, actives are tested in a secondary screen that assays for penetration of *Magnaporthe grisea* into plant tissue such as onion epidermal tissue. Positive test samples incubated with epidermal pieces inhibit penetration of the hyphae arising from the melanized penetration structure, the appressorium.

### Detailed Description of the Invention

This invention takes advantage of the distinctive color changes which occur when melanin biosynthesis is blocked in fungi that synthesize the pigment. The screen detects compounds active at any step of the melanin biosynthesis pathway.

Fungi producing melanin can be employed in the screening test of this invention. Particularly preferred are fungi that produce 1,8-dihydroxynaphthalene (DHN) melanin. At least 50 fungi synthesize DHN melanin. These include, for example, species of Ascomycotina and related Deuteromycotina such as *Cochliobolus carbonum, C. miyabeanus, Pleospora infectoria, Sclerotinia minor, S. trifoliorum, Whetzelinia sclerotiorum, Alternaria alternata, A. brassicicola, Aureobasidum pullulans, Botrytis cinerea, Cladosporium bantianum, C. carrionii, Colletotrichum gossypii, C. lagenarium, C. lindemuthianum, Curvularia protuberata, Diplodia gossypina, Diplodia natalensis, Drechslera sorokiniana, Exophiala jeanselmei, E. werneckii, Fonsecaea compacta, F. pedrosoi, Macrophomina phaseoli, Microdochium bolleyi, Phialophora richardsiae, P. verrucosa, Pyricularia oryzae, Rhizoctonia leguminicola, Sclerotium cepivorum, Thielaviopsis basicola, Verticillium albo-atrum, V. dahliae, V. nigrescens, V. tricorpus, and Wangiella dermatitidis.* It is an advantage of the invention that melanin-producing fungi are readily available, isolated from natural sources or obtained as fresh cultures, as frozen cultures with or without cryoprotectants such as dimethylsulfoxide, as plugs of these, or as conidia.

Preferred fungi of this type pigment in the light or in the dark. *Magnaporthe grisea* is preferred in one embodiment. Because of ease of growth under laboratory conditions, producing a highly uniform conidiating lawn of growth in plated cultures, *Cladosporium cucumerinum* is especially preferred. The fungus pigments in the light or in the dark.

Any type of solidified or liquid medium that will support growth and reproduction of melanin-containing fungi may be employed as growth medium in the method of this invention. Numerous fungal media are known to the skilled artisan, and include standard mycological media, for example, potato dextrose media (PDM), potato dextrose agar (PDA) media, oatmeal agar media, Misato-Hara media containing soluble starch, yeast extract, and bactoagar, basal growth media (BGM) containing glucose, vitamins, minerals, and water, and the like. Antibacterials that do not inhibit fungal cell growth such as chloramphenicol may be added to the media to reduce contamination and facilitate culturing. Preferred media do not contain chromogenic or chromophoric ingredients that mask color changes and interfere with the sensitivity of the screen; therefore, media containing oatmeal are not preferred. Solidified media are preferred; potato dextrose agar media is preferred in one embodiment. The fungus can be incorporated into the agar prior to pouring and incubation.

In the practice of this invention, test samples are incubated in cultures of any fungal species that produces melanin. A known melanin synthesis inhibitor is employed as a positive control. Potentially active agents are identified by the observation of altered pigmentation in the presence of test sample.

A preferred method comprises adding a test sample to a *Cladosporium cucumerinum* culture. The test sample is introduced to a disk or a well on a culture plate in a standard diffusion assay using solidified media, or introduced into one of a series of equivalent tissue culture tubes or bottles in a standard turbidity assay using liquid media. The culture is incubated for such time under such conditions sufficient to observe cell growth and pigmentation in a corresponding culture or culture plate area containing no test sample. Pigmentation in the culture containing or surrounding the test sample is compared with pigmentation in the culture or culture area containing no test sample. The presence of melanin synthesis inhibition is determined by observing alterations in pigmentation. In a culture plate, this is a zone of color tones surrounding the test sample. In a culture tube series, this is color tone differences observed visually or spectrophotometrically.

Preferred methods employ a known melanin synthesis inhibitor such as pyroquilon or tricyclazole as a positive control; tricyclazole is preferred. The control is useful in discerning whether the screen is functioning properly and in identifying positives by direct comparison. In a disk or well diffusion assay, the control is introduced to a disk or a well in the culture plate at the same time the test sample is introduced. After the incubation period, pigmentation in the vicinity of the control is compared to pigmentation in the vicinity of the test sample. A test sample that inhibits melanin biosynthesis exhibits altered pigmentation as compared to the rest of the culture even if the color change is not the same as that observed in the vicinity of the positive control. Likewise, in a turbidity assay, the control is introduced into an equivalent tissue culture tube or bottle and incubated with test and negative cultures; after incubation, cultures containing a test sample that inhibits melanin biosynthesis exhibit different pigmentation from that observed in tubes containing fungus only.

Preferred screening tests employ solidified cultures in transparent plates or dishes. Pigmentation is ordinarily observed visually as circular zones of color around disks or wells in these cultures. The color changes are observed by viewing the upper or undersurface of the plate; viewing the undersurface is preferred. In an olive-green lawn of wild-type *Cladosporium cucumerinum*, for example, the colors are generally tones of white, tan, red, or mixtures of these. Inhibition of fungal growth is seen with some test samples; in these cases, color change in positive samples is observed as a zone surrounding the circle of growth inhibition.

A distinct advantage of the invention is its speed and simplicity. Fungi containing melanin are readily available. Using solidified media in culture plates, the protocol is extremely simple. Many samples are readily analyzed in a short time for the identification of agents potentially useful as fungicides.

It is another advantage of the invention that small amounts of biochemical or chemical agents are required in the test. In a standard assay, for example, which employs solidified media in a plate, as little as 250 ng of a biochemical or chemical test sample is applied to a disk or in a well. For fermentation broths, little or no concentration is necessary, but may be required for some samples. As a positive control, as little as 200 ng of tricyclazole is required.

It is a further advantage of the invention that the melanin biosynthesis inhibition assay is a low positive rate assay, so that secondary tests are not of crucial importance. However, secondary screens may be employed to prioritize actives found using the screen. A preferred secondary screen assays for penetration of hyphae from appressoria of the fungus *Magnaporthe grisea* into plant tissue such as onion epidermal tissue. Initial stages of infection of rice leaf host are mimicked in the assay. About 90 to 95% of wild-type *M. grisea* conidia that germinate result in penetration of freshly harvested onion epidermis, whereas no penetration is observed with albino or buff mutants and no penetration of wildtype is observed in the presence of the known melanin biosynthesis inhibitor, tricyclazole.

In the practice of the secondary screening method, epidermal onion pieces are incubated with test sample in the presence of *M. grisea* conidia for such time under such conditions sufficient to observe germination, appressorial formation and hyphal penetration in the absence of sample. Tricyclazole is used as a positive control in some embodiments. Positive test samples are identified by observation of penetration inhibition, using standard methodology such as microscopy. The screen is rapid, yielding results in 24 hours, and simple to perform.

Standard *in vivo* fungicide discovery screens are employed in some embodiments as tertiary tests to further prioritize actives. In typical screens, a variety of phyto-pathogenic fungi are used to infect plants treated with test compounds. Active compounds block or reduce the appearance of disease symptoms.

The following examples are presented to further illustrate and explain the present invention and should not be taken as limiting in any regard.

### Example 1

In order to determine the efficacy of a screen for melanin biosynthesis according to this invention, a large number of compounds are tested, including known antifungal and antibacterial compounds selected to represent diverse mechanisms of action.

Components of the test media are first prepared using analytical grade or cell culture tested reagents obtained from the sources indicated.

Potato dextrose agar (PDA) media for routine culturing of *Cladosporium cucumerinum* is prepared by dissolving

| | |
|---|---|
| Difco Potato Dextrose Agar | 39 g |
| and Supplemental Bactoagar | 1 |
| in Distilled Water | to 1 liter |

and heating to near boiling while stirring continuously. The mixture is then autoclaved at 121°C for 25 minutes. Plates are poured and stored at 4°C.

Potato dextrose medium (PDM) for the screening test is prepared by melting

| | |
|---|---|
| Type VII Sigma Agarose and adding | 17.5 g |
| Difco Potato Dextrose Broth | 24 g |
| Distilled Water | to 1 liter. |

The mixture is autoclaved as 150 ml aliquots at 121°C for 25 minutes.

A chloramphenicol stock solution is prepared to contain 15 mg chloramphenicol per mL absolute ethanol and is stored at -20°C.

Conidia from *Cladosporium cucumerinum*, 10⁷/mL in 0.03% Tween 20™, retain their viability for at least 12 weeks of storage in 1.8 ml cryovials at -85°C. Inoculum for running the screen are raised once every three months and stored without a cryoprotectant at the working concentration of 10⁷ conidia/mL.

Fresh inoculum suspensions are prepared from conidial suspensions stored at -85°C. A cryovial is thawed by hand-heat, and 200 uL spread on the surface of PDA in 9 cm Petri dishes. Plates are placed in a resin box (to prevent fungal escape) and incubated at room temperature, 40 cm below laboratory fluorescent lights. Seven days later conidia are collected. Five mL of 0.03% Tween 20™ is added to each plate, and the conidia are released by rubbing the mycelium with a glass rod. The suspension is passed through a layer of sterile Miracloth™ and the concentration adjusted to 10⁷ conidia/mL in 0.03% Tween 20™ using a haemocytometer. Aliquots of 1 mL are dispensed into cryovials and placed in a polystyrene holder to freeze slowly to -85°C.

For long-term storage of the fungal line, 6 mm diameter mycelial plugs are cut with a cork-borer from a 7-day plate. Plugs are stored in cryovials, 5 plugs/mL of 15% glycerol. The cryovials are placed in a polystyrene holder and frozen slowly to -85°C. For culturing, plugs are warmed by hand-heat before transferring pieces of mycelial plug to PDA.

For the test, 150 mL of PDM is cooled to 35°C, and 0.1 mL of chloramphenicol stock solution is added by gentle swirling. A vial of *C. cucumerinum* conidial suspension (10⁷ conidia/ml in Tween 20™) thawed with hand-heat is mixed into the growth medium using gentle swirling. The mixture is poured immediately into a Biotray, and the agar is allowed to solidify.

Test compounds are applied to 0.63 cm (¼ inch) filter disks. Each test includes a positive control, 200 ng tricyclazole in 20% dimethyl sulphoxide (DMSO). The plate is incubated at room-temperature (∼23°C) 40 cm below laboratory fluorescent lights. The plate is read 40 hours later. Active compounds show normal mycelial growth as seen by viewing the upper surface of the plate, but altered pigmentation on the undersurface of the plate. Compared to the olive-green color of wild-type *C. cucumerinum*, colors exhibit white, tan, or red tones. The Biotrays are autoclaved before disposal.

A panel containing varied antifungal types is tested using the above-described melanin biosynthesis inhibition screen of this invention using a disk diffusion assay (Table 1, below, listed with results). The compounds are applied as 20 *u*l droplets from a 1 mg/ml stock solution. No positives are found.

**TABLE 1:**

| Antifungal Panel | |
|---|---|
| *Compound* | *Observation* |
| Roundup® | inhibits, 15 mm radius cleared |
| Phosphenothricin | inhibits, 20 mm radius cleared |
| Ketoconazole | inhibits, 26 mm radius cleared |
| Amphotericin B | no effect on growth or pigmentation |
| Cerulenin | fungal thinning, 25 mm radius |
| Haloprofin | inhibits, 13 mm radius cleared |
| Noconazole | inhibits, 8 mm radius cleared |
| Miconazole | inhibits, 31 mm radius cleared |
| Cycloheximide | inhibits, 22 mm radius cleared |
| Polyoxin D | inhibits, 7 mm radius cleared |
| Nikkomycin | inhibits, 38 mm radius cleared |
| Diniconazole | inhibits, 27 mm radius cleared |
| Fenarimole | inhibits, 22 mm radius cleared |
| Maneb | no effect on pigmentation, growth thin |
| Metalaxyl | no effect on growth or pigmentation |
| Ronilon | very slight effect on growth |
| Tridemorph | inhibits, 6 mm radius cleared |
| Kanamycin | inhibits, 10 mm radius cleared |
| Benomyl | inhibits, 31 mm radius cleared |
| Tunicamycin | inhibits, 22 mm radius cleared |
| Cyanobutyrate | no effect on growth or pigmentation |
| Prowl® | no effect on growth or pigmentation |
| Carboxin | inhibits, 21 mm radius cleared |
| Antimycin | inhibits, 13 mm radius cleared |
| Tolnaftate | inhibits, 13 mm radius cleared |
| 5-Fluoro-Cytosine | inhibits, 35 mm radius cleared |
| Econazole | inhibits, 32 mm radius cleared |
| Sulfometuron methyl | inhibits, 6 mm radius cleared |
| Amino-triazole | inhibits, 13 mm radius cleared |
| U-18666 | no effect on growth or pigmentation |

### Example 2

The screen of Example 1 is used to test randomly selected antibiotics (including the 42 listed with results in Table 2 below) in a panel of 139 compounds that include natural products. No actives are found, but the orange or yellow color of some compounds impart color as they diffuse.

**TABLE 2:**

| *C. cucumerinum* Antibiotic Panel | |
|---|---|
| *Compound* | *Observation* |
| Anisomycin | no effect on growth or pigmentation |
| Anthelmycin | inhibits, 14 mm radius cleared |
| A8363 | fungal thinning, 4 mm radius |
| AD97 | no effect on growth or pigmentation |
| AN272 Alpha | no effect on growth or pigmentation |
| BL580 Zeta | no effect on growth or pigmentation |
| BM123 Alpha Sulfate | no effect on growth or pigmentation |
| BM782 Epsilon | no effect on growth or pigmentation |
| Ascotoxin | no effect on growth or pigmentation |
| Aureofungin | no effect on growth or pigmentation |
| Azalomycin | inhibits, 14 mm radius cleared |
| Dermostatin | no effect on growth or pigmentation |
| Destomycin A | inhibits, 18 mm radius cleared |
| Flavofungin | no effect on growth or pigmentation |
| Folimycin | inhibits, 4 mm radius cleared |
| Gliotoxin | inhibits, 17 mm radius cleared |
| Hamycin | no effect on growth or pigmentation |
| Monicamycin | no effect on growth or pigmentation |
| Mycolutein | fungal thinning, 16 mm radius |
| Naramycin B | inhibits, 15 mm radius cleared |
| Neohumidin | no effect on growth or pigmentation |
| Neomycin Sulfate | no effect on growth or pigmentation |
| Nybomycin | no effect on growth or pigmentation |
| Pentamycin | no effect on growth or pigmentation |
| Picromycin | no effect on growth or pigmentation |
| Protomycin/Streptimidone | no effect on growth or pigmentation |
| Puromycin Aminonucleoside | no effect on growth or pigmentation |
| Pyrenophorin | inhibits, 12 mm radius cleared |
| Ramulosin | no effect on growth or pigmentation |
| Ristocedin | no effect on growth or pigmentation |
| Rugulosin | inhibits, 9 mm radius cleared |
| Sparsomycin | no effect on growth or pigmentation |
| Streptomycin Sulfate | no effect on growth or pigmentation |
| Sulfocidin | fungal thinning, 5 mm radius |
| Tennecetin | no effect on growth or pigmentation |
| Tetrahydro Spiramycin Base | no effect on growth or pigmentation |
| Thioaurin | inhibits, 16 mm radius cleared |
| Thiolutin | inhibits, 23 mm radius cleared |
| Trichomycin | no effect on growth or pigmentation |
| Tubercidin | fungal thinning, 10 mm radius |
| Tylosin Tartrate | no effect on growth or pigmentation |
| Vancomycin HCl | no effect on growth or pigmentation |

### Example 3

The screen of Example 1 is employed to test the antifungal panel listed in Table 1, except that *Magnaporthe grisea* is used as the culture fungus. No actives are found.

The screen using *M. grisea* is also employed to test 75 randomly selected antibiotics (listed in Table 3 below) No actives are found (but the orange or yellow compounds impart color as they diffuse).

**TABLE 3:**

| *M. grisea* Antibiotic Panel | |
|---|---|
| Pimaricin | Hamycin |
| Streptogramin Type AA11 | Carbomycin |
| Monazomycin | Frenolicin |
| Nystatin | Fusarinic Acid |
| Aspartocin | Netropsin |
| Bacitracin | Tylosin Tartrate |
| Clavacin | B02964 Complex |
| Citrinin | Chloramphenical |
| Avoparcin Sulfate | Alazopeptin |
| Isoquinocycline HCl | A8363 |
| Neutramcin | Actinomycin Crude |
| A1531 | BM123 Gamma HCl |
| Leucomycin | AD97 |
| Actithiazic Acid | Phenazine a-COOH |
| A0341 Beta | Paromomycin Sulfate |
| Angustmycin A and C | Streptomycin Sulfate |
| Gliotoxin | Tennecetin |
| Gibberellic Acid | A4825 |
| Puromycin HCl | BO2964 Complex |
| Puromycin Aminonucleoside | Nucleocidin |
| BM123 Alpha Sulfate | Nonactin |
| Etamycin (Na Salt) | Valinomycin |
| Mocimycin | C19004 Complex |
| Neomycin Sulfate | Avilamycin |
| Viomycin Sulfate | V214W |
| Netropsin | V214X |
| Lincomycin HCl | Vanomycin HCl |
| Picromycin (Free Base) | Ristocetin |
| A9537 | Relomycin |
| AN272 Alpha | C08078 a |
| Levomycin | Blasticidin S |
| AM374#22 | BL580 Alpha |
| Antiprozoin | Declomycin HCl |
| BL580 Zeta | Usnic Acid |
| Lincomycin Complex | Geldanamycin |
| Z1220A | Isoquinocycline HCl |
| AA575 Gamma | BM782 Epsilon |
| 4-Dedimethylamino-4-methylamino-anhydrotetracycline | |
| Tetrahydro Spiramycin Base | |

### Example 4

A secondary screen for melanin biosynthesis inhibitors that is used to screen positives from the primary screen of Example 1 is described in this example.

Onion rings from freshly harvested onions (grown from Burpee Yellow Globe seed #5276-1) are dipped in chloroform for 3 seconds and then rinsed in 2 liters sterile water. Epidermal pieces are floated onto water contained in either a Petri dish or an untreated 48-cell well culture plate (depending on the number of samples to be checked) . A 10 ul droplet of either a water control or the test compound is applied to the surface. A small number of conidia of a wild-type strain *M. grisea* are then incorporated into the droplet with a pipette tip in 0.5 ml water or test compound. After incubation at 25°C for 24 hours in the presence or absence of light, the whole epidermal pieces are examined directly using differential interference contrast light microscopy. Presence of penetration, observed with the water control, indicates a non-inhibitory compound. Absence of penetration indicates a positive test sample.

Using this procedure, the presence of 15 ng tricyclazole in 1.5% acetone results in 100% inhibition of penetration. Acetone is itself non-inhibitory even at 4% when applied to the epidermal surface with water underneath. However, only 2% acetone can be used if applied to both sides of the epidermis.

When the assay method is repeated using albino or buff melanin-deficient mutants of *M. grisea* instead of the wildtype, the conidia germinate but no penetration is observed and fungal growth is inhibited.

The above description is for the purpose of teaching the person of ordinary skill in the art of how to practice the present invention, and it is not intended to detail all those obvious modifications and variations of it which will become apparent to the skilled worker upon reading the description.

### BIBLIOGRAPHY

Bell, A.A., and Wheeler, M.H., *Ann. Rev. Phytopathol.* 24: 411-51 (1986).

## Claims

1. A method for screening for the presence or absence of melanin synthesis inhibition by a test sample which comprises:
(a) preparing a solidified culture of a fungus that produces melanin;
(b) adding said test sample to the culture;
(c) incubating said test sample in the culture and a first corresponding culture containing no test sample and a second corresponding culture containing a known melanin synthesis inhibitor as a control for such time under such conditions sufficient to observe cell growth and pigmentation in the first corresponding culture containing no test sample; and
(d) determining the presence of melanin synthesis inhibition by visual observation that pigmentation in the solidified culture with the test sample is altered in comparison to pigmentation in the first corresponding culture containing no test sample.

2. A method according to claim 1 wherein the culture containing test sample, the culture containing no test sample, and the culture containing a known melanin synthesis inhibitor comprise different portions of a single solidified fungal culture.

3. A method according to claim 1 wherein the known melanin synthesis inhibitor is selected from the group consisting of pyroquilon and tricyclazole.

4. A method according to claim 2 wherein the fungus that produces melanin is selected from the group consisting of *Magnaporthe* grisea and *Cladosporium cucumerinum*.

5. A method according to claim 1 wherein the fungus produces dihydroxynaphthalene melanin.

6. A method for the secondary screening of a test sample that inhibits melanin synthesis in the method according to claim 5 comprising assaying for inhibition of penetration of germinated conidia by way of the appressoria into plant tissue by said sample.

7. A method according to claim 6 wherein the conidia are *Magnaporthe grisea* conidia and the plant tissue is onion epidermis tissue.

8. A method for screening for the presence or absence of dihydroxynaphthalene melanin synthesis inhibition by a chemical or biochemical test sample which comprises:
(a) preparing a solidified culture of *Cladosporium cucumerinum*;
(b) adding to said culture on a disk or in a well a chemical or biochemical test sample;
(c) adding to said culture on a disk or in a well the known melanin synthesis inhibitor tricyclazole as a control;
(d) incubating said culture for such time under such conditions sufficient to observe fungal cell growth and pigmentation;
(e) comparing pigmentation in the culture in the vicinity of test sample with pigmentation in the vicinity of the control and with pigmentation in the rest of the culture; and
(f) determining the presence of said melanin synthesis inhibition by visual observation that pigmentation in the vicinity of test sample and in the vicinity of the control differs from pigmentation in the rest of the culture.

9. A method according to claim 8 wherein said culture contains potato dextrose media and chloramphenicol.

## Patentansprüche

1. Verfahren zum Screenen auf das Vorhandensein bzw. Fehlen der Melaninsynthesehemmung durch eine Testprobe, umfassend die Schritte:
(a) Herstellen einer verfestigten Kultur eines melaninproduzierenden Pilzes;
(b) Zugeben der Testprobe zu der Kultur;
(c) Inkubieren der Testprobe in der Kultur sowie einer ersten Parallelkultur, die keine Testprobe enthält, und einer zweiten Parallelkultur, die einen bekannten Melaninsynthesehemmstoff als Kontrolle enthält, über einen Zeitraum und unter Bedingungen, der bzw. die ausreichen, daß Zellwachstum und Pigmentierung in der ersten Parallelkultur ohne Testprobe beobachtet werden können, sowie
(d) Nachweisen des Vorhandenseins von Melaninsynthesehemmung durch die visuelle Beobachtung, daß die Pigmentierung in der verfestigten Kultur mit der Testprobe im Vergleich zu der Pigmentierung in der ersten Parallelkultur ohne Testprobe verändert ist.

2. Verfahren nach Anspruch 1, bei dem die Kultur mit der Testprobe, die Kultur ohne Testprobe und die Kultur, die einen bekannten Melaninsynthesehemmstoff enthält, unterschiedliche Teile einer einzigen verfestigten Pilzkultur umfassen.

3. Verfahren nach Anspruch 1, bei dem der bekannte Melaninsynthesehemmstoff aus der Gruppe Pyroquilon und Tricyclazol stammt.

4. Verfahren nach Anspruch 2, bei dem der melaninproduzierende Pilz aus der Gruppe *Magnaporthe grisea* und *Cladosporium cucumerinum* stammt.

5. Verfahren nach Anspruch 1, bei dem der Pilz Dihydroxynaphthalinmelanin produziert.

6. Verfahren für das sekundäre Screenen einer Testprobe, die in dem Verfahren nach Anspruch 5 die Melaninsynthese hemmt, bei dem man die Hemmung der Penetration von gekeimten Konidien in pflanzliches Gewebe mittels der Appressorien durch diese Probe in einem Assay prüft.

7. Verfahren nach Anspruch 6, wobei es sich bei den Konidien um Konidien von *Magnaporthe grisea* und bei dem pflanzlichen Gewebe um Zwiebelepidermisgewebe handelt.

8. Verfahren zum Screenen auf das Vorhandensein oder Fehlen der Dihydroxynaphthalinmelaninsynthesehemmung durch eine chemische oder biochemische Testprobe, umfassend die folgenden Schritte:
(a) Herstellen einer verfestigten Kultur von *Cladosporium cucumerinum*;
(b) Zugabe einer chemischen oder biochemischen Testprobe zu dieser Kultur auf einer Scheibe oder in einem Well;
(c) Zugabe des bekannten Melaninsynthesehemmstoffs Tricyclazol zu der Kultur auf einer Scheibe oder in einem Well als Kontrolle;
(d) Inkubieren der Kultur über einen Zeitraum und unter Bedingungen, der bzw. die ausreichen, um Pilzzellenwachstum und Pigmentierung zu beobachten;
(e) Vergleichen der Pigmentierung in der Kultur in der Umgebung der Testprobe mit der Pigmentierung in der Umgebung der Kontrolle und mit der Pigmentierung im Rest der Kultur; sowie
(f) Nachweisen des Vorhandenseins von Melaninsynthesehemmung durch die visuelle Beobachtung, daß sich die Pigmentierung in der Umgebung der Testprobe und in der Umgebung der Kontrolle von der Pigmentierung im Rest der Kultur unterscheidet.

9. Verfahren nach Anspruch 8, wobei die Kultur Kartoffeldextrosemedien und Chloramphenicol enthält.

## Revendications

1. Procédé de triage pour détecter la présence ou l'absence d'inhibition de la synthèse de mélanine par un échantillon d'essai, qui consiste à :
(a) préparer une culture solidifiée d'un champignon qui produit de la mélanine ;
(b) ajouter ledit échantillon d'essai à la culture ;
(c) mettre en incubation ledit échantillon d'essai dans la culture et une première culture correspondante ne contenant pas d'échantillon d'essai et une seconde culture correspondante contenant un inhibiteur connu de la synthèse de mélanine comme témoin pendant un temps et dans des conditions qui sont suffisants pour observer une croissance cellulaire et une pigmentation dans la première culture correspondante ne contenant pas d'échantillon d'essai ; et
(d) déterminer la présence d'inhibition de la synthèse de mélanine en observant visuellement que la pigmentation dans la culture solidifiée contenant l'échantillon d'essai est altérée comparativement à la pigmentation dans la première culture correspondante ne contenant pas d'échantillon d'essai.

2. Procédé selon la revendication 1, dans lequel la culture contenant l'échantillon d'essai, la culture ne contenant pas d'échantillon d'essai et la culture contenant un inhibiteur connu de la synthèse de mélanine sont constituées par des portions différentes d'une même culture fongique solidifiée.

3. Procédé selon la revendication 1, dans lequel l'inhibiteur connu de la synthèse de mélanine est choisi dans la classe formée par le pyroquilon et le tricyclazole.

4. Procédé selon la revendication 2, dans lequel le champignon qui produit de la mélanine est choisi dans la classe formée par *Magnaporthe grisea* et *Cladosporium cucumerinum*.

5. Procédé selon la revendication 1, dans lequel le champignon produit de la dihydroxynaphtalène-mélanine.

6. Procédé pour le triage secondaire d'un échantillon d'essai qui inhibe la synthèse de mélanine dans le procédé selon la revendication 5, consistant à déterminer l'inhibition par ledit échantillon de la pénétration de conidies germées au moyen des appressoria dans un tissu végétal.

7. Procédé selon la revendication 6, dans lequel les conidies sont les conidies de *Magnaporthe grisea* et le tissu végétal est du tissu épidermique d'oignon.

8. Procédé de triage pour détecter la présence ou l'absence d'inhibition de la synthèse de dihydroxynaphtalène-mélanine par un échantillon d'essai chimique ou biochimique, qui consiste à :
(a) préparer une culture solidifiée de *Cladosporium cucumerinum ;*
(b) ajouter à ladite culture sur un disque ou dans un puits un échantillon d'essai chimique ou biochimique ;
(c) ajouter à ladite culture sur un disque ou dans un puits du tricyclazole comme inhibiteur connu de la synthèse de mélanine à titre de témoin ;
(d) faire incuber ladite culture pendant un temps et dans des conditions suffisants pour observer une croissance cellulaire fongique et une pigmentation ;
(e) comparer la pigmentation dans la culture au voisinage de l'échantillon d'essai avec la pigmentation au voisinage du témoin et avec la pigmentation dans le reste de la culture ; et
(f) déterminer la présence de ladite inhibition de la synthèse de mélanine en observant visuellement que la pigmentation au voisinage de l'échantillon d'essai et au voisinage du témoin diffère de la pigmentation dans le reste de la culture.

9. Procédé selon la revendication 8, dans lequel ladite culture contient un milieu au dextrose-pomme de terre et du chloramphénicol.
